# EUROPÄISCHE PATENTSCHRIFT

(11) **EP 2 222 231 B1**
(45) Veröffentlichungstag und Bekanntmachung des Hinweises auf die Patenterteilung: **27.02.2019**
(21) Anmeldenummer: 08802410.4
(22) Anmeldetag: 19.09.2008
(51) Int. Cl.: A61B 17/04, A61F 2/08

(54) **VORRICHTUNG ZUR IMPLANTATION UND EIN SYSTEM FÜR EINE BEANSPRUCHUNG EINES KREUZBANDS IN EINEM KNIEGELENK**
DEVICE FOR IMPLANTATION, AND A SYSTEM FOR LOADING A CRUCIATE LIGAMENT IN A KNEE JOINT
DISPOSITIF D'IMPLANTATION, ET SYSTÈME PRÉVU EN RÉPONSE À UNE SOLLICITATION D'UN LIGAMENT CROISÉ DANS UNE ARTICULATION DU GENOU

(30) Priorität: 27.12.2007 DE 102007062749; 01.04.2008 DE 102008016607
(43) Veröffentlichungstag der Anmeldung: 01.09.2010
(73) Patentinhaber: Mathys AG Bettlach, 2544 Bettlach (CH)
(72) Erfinder: EGGLI, Stefan, CH-3007 Bern (CH)
(74) Vertreter: Körfer, Thomas
(86) Internationale Anmeldenummer: PCT/EP2008/007903
(87) Internationale Veröffentlichungsnummer: WO 2009/083047

(56) Entgegenhaltungen:
- WO-A-00/13601
- WO-A-02/38059
- WO-A1-97/36557
- DE-U1- 8 914 308
- GB-A- 2 078 528
- US-A- 5 458 601
- US-A- 5 507 812
- US-A- 6 036 694
- US-A1- 2004 098 050
- US-A1- 2005 222 488

## Beschreibung

Die Erfindung bezieht sich auf eine Vorrichtung zur Implantation in einen Knochen und auf ein System für eine kontrollierte Beanspruchung des rekonstruierten vorderen Kreuzbandes, wobei die erfindungsgemäße Vorrichtung zur Implantation im erfindungsgemäßen System integriert ist.

Das menschliche Kniegelenk wird durch das vordere Kreuzband und das hintere Kreuzband im Kniegelenksinnenraum stabilisiert. Bei einem Verdrehtrauma des Kniegelenks werden diese beiden Bänder sehr oft überbelastet bis es zur Ruptur bzw. zu einem Abreißen kommt. Dabei ist das vordere Kreuzband ca. 9-mal häufiger betroffen als das hintere Kreuzband. Sämtliche konservativen Therapieversuche oder Versuche das vordere Kreuzband zu nähen, sind mit erheblichen Problemen behaftet. Aus diesem Grund wird bei persistierender Instabilität des verletzten Kniegelenks gemäß dem Stand der Technik das vordere Kreuzband entfernt und die Kniegelenkstabilität durch ein Transplantat aus Sehnenmaterial, entweder der Patallarsehne, der Semitendinosussehne oder der Quadricepssehne, wiederhergestellt. Ein Nachteil dieser Methode besteht darin, dass diese Bandstruktur avital ist, also keine Sensibilität mehr aufweist und mit zunehmender Dauer wieder an Stabilität verliert.

Die deutsche Offenlegungsschrift DE 38 03 208 A1 beschreibt eine Vorrichtung für die Wiederherstellungschirurgie, wobei das vordere Kreuzband im menschlichen Kniegelenk permanent wiederhergestellt wird. Dabei wird das Schienbein relativ zum Oberschenkelknochen stabilisiert und dem Knie ein voller Bewegungsbereich wiedergegeben, indem ein Ersatzband innerhalb des Kniegelenks durch präzises örtliches Festlegen der Enden und der Winkellage so eingelegt wird, dass bei einer Bewegung des Kniegelenks das Ersatzband keine Längenänderung erfährt (isometrische Bewegung) oder dass die Längenänderung des Ersatzbandes der physiometrischen Bewegung eines natürlichen Kreuzbandes entspricht. Der Nachteil bei dieser Vorrichtung besteht darin, dass das vordere Kreuzband im menschlichen Kniegelenk durch ein Ersatzband, welches ebenfalls aus einem Transplantat besteht, permanent wiederhergestellt wird. Dabei wird das beschädigte, natürliche Band endgültig aus dem Kniegelenk entfernt, wobei das künstliche Ersatzband dessen Funktion nur unzureichend übernimmt.

Die US Patentschrift 5,702,422 beschreibt ein Verfahren zur Reparatur von Rissen und Rupturen eines vorderen Kreuzbands eines menschlichen Knies, wobei das Band innerhalb der Spalte zwischen den beiden Kondylen des Femurknochens mittels eines Bolzens für die Wundnaht an seinem antomischen Ansatz wieder verbunden wird. Dabei wird die Heilung am Ansatz dadurch beschleunigt, dass in der Spongiosa eine Vertiefung zum Auffangen von Blut ausgebildet ist.

Der Nachteil dieses Verfahrens besteht darin, dass die Wundnaht mit dem Bolzen verbunden ist, wobei permanent ein Zug auf das eingerissene bzw. auf das durchgerissene Band ausgeübt wird, so dass das zusammengewachsene Band länger ist als das ursprüngliche gesunde Band und dadurch in seiner Funktionalität beeinträchtigt ist.

Dokument WO 97/36557 A1 offenbart ein Befestigungselement zur Verbindung einer Sehne mit einem Knochenteil eines menschlichen oder tierischen Körpers, wobei das Befestigungselement ein hohles, im wesentlichen zylindrisches Element aufweist, welches in der durchgehenden Öffnung eines Knochenteils festlegbar ist und ein Eingriffselement, mit dem die Sehne in dem zylindrischen Element an wenigstens zwei unterschiedlichen Axialstellungen verankerbar ist. Das Dokument WO 97/36557 A1 lehrt auch, dass der Außenkörper in seinem Inneren eine Dämpfungseinrichtung zwischen einem distalen und einem proximalen Ende aufweist, wobei innerhalb der Dämpfungseinrichtung ein Befestigungselement angeordnet ist, zur Fixierung von Fasern, wobei die Fasern mittels Verklemmung an dem einen Befestigungselement befestigbar sind und das Befestigungselement als teilweise Kegel mit einer glatten Mantelfläche ausgeführt ist. Dokument WO 97/36557 A1, Figur 3, zeigt auch eine Spannhülse, die eine Stirnfläche aufweist, die auf eine Spiralfeder drückt, wobei die Spiralfeder flach auf der Stirnseite aufliegt und wobei die Spiralfeder an der Innenwand des Eingriffselements geführt wird.

Weitere sachgemäße Vorrichtungen und Systeme werden zum Beispiel in US 5 507 812 A, GB 2 078 528 A, US 6 036 694 A, WO 00/13601 und WO03/38059 beschrieben.

Der Erfindung liegt die Aufgabe zu Grunde, eine Vorrichtung und ein System für eine temporäre Entlastung eines rekonstruierten natürlichen vorderen Kreuzbands im menschlichen Kniegelenk bzw. für eine beliebige Bandstruktur eines menschlichen oder tierischen Gelenks anzugeben.

Bezüglich der Vorrichtung bzw. des Systems mit der Vorrichtung wird die genannte Aufgabe erfindungsgemäß gelöst durch die Merkmale des Anspruchs 1 bzw. 14. Vorteilhafte Weiterbildungen sind Gegenstand der hierauf rückbezogen Unteransprüche.

So umfasst die erfindungsgemäße Vorrichtung zur Implantation in einen Knochen einen mit einem Außengewinde versehenen Außenkörper, welcher eine Stirnseite und einen Boden aufweist. In seinem Inneren weist der Außenkörper eine Dämpfungseinrichtung zwischen einem distalen Ende und einem proximalen Ende auf, wobei innerhalb der Dämpfungseinrichtung zumindest ein Befestigungselement angeordnet ist, welches zumindest einen Faden fixiert, der am proximalen Ende der Vorrichtung durch eine Aussparung im Boden aus dem Außenkörper heraus geführt ist.

Der Erfindung liegt das Konzept zugrunde, dass jedes Band des menschlichen Körpers weitreichend eine Selbstheilungstendenz aufweist. So werden zum heutigen Zeitpunkt fibulotalare Bandrupturen oder AC-Gelenksrupturen praktisch alle konservativ behandelt. Selbst der Riss der großen Achillessehne wird an vielen Zentren mittlerweile konservativ behandelt. Wie bereits erwähnt, hat die konservative Behandlung des vorderen Kreuzbands in der Vergangenheit versagt. Wohl kam es zu einer Vernarbung des Bandes, die Instabilität des Kniegelenks persistierte jedoch. Die stabilisierende Wirkung dieses Bandes geht verloren, da ein Kniegelenk zur Heilung des Bandapparates nicht ruhig gestellt werden kann. Das vordere Kreuzband braucht zur Primärheilung ca. 6 Wochen. Eine Ruhigstellung für diese Zeit ist absolut unmöglich, da durch eine derartige Immobilisierung das Kniegelenk praktisch steif würde. Bewegt man aber das Kniegelenk in der Heilungsphase bei persistierender Instabilität, so kommt es bei jeder zunehmenden Flexion zu einer anteroposterioren Translation von bis zu 10 mm des Kniegelenks. Diese translatorische Instabilität wird bei der Selbstheilung des Bandes quasi miteingebaut. Das Band kann nicht auf die ursprüngliche Länge zusammenheilen, sondern verlängert sich entsprechend und erfüllt demnach die stabilisierende Funktion nicht mehr.

Die mit der erfindungsgemäßen Vorrichtung zur Implantation in einen Knochen erzielten Vorteile bestehen insbesondere darin, das Kniegelenk während der Selbstheilungsphase in jeder Flexionsstellung genau in der richtigen anteroposterioren Translation zu stabilisieren. Die erfindungsgemäße Vorrichtung, welche als isotonische Kniegelenksschraube konzipiert ist und welche ein integraler Bestandteil des erfindungsgemäßen Systems ist, übernimmt während der Heilungsphase quasi die Gelenk-Stabilisierung, die vorher das natürliche, körpereigene und unversehrte vordere Kreuzband inne hatte. Durch die isotonische Kniegelenksschraube bzw. durch das erfindungsgemäße System mit der erfindungsgemäßen Vorrichtung wird der Unterschenkel gegenüber dem Oberschenkel dauernd in eine hintere Schublade gezogen.

Dadurch werden die zwei gerissenen Faserbündel des vorderen Kreuzbandes einander auf eine kürzest mögliche Distanz angenähert. Vorteilhafterweise können die beiden Bandstümpfe mit Hilfe des erfindungsgemäßen Systems ohne die verlorengegangene Stabilität wieder in ursprünglicher Position und ursprünglicher Länge zusammenheilen und somit später ihre ursprüngliche Funktion, insbesondere die Stabilisierung des Gelenks wieder vollständig erfüllen.

Des Weiteren ist Dämpfungseinrichtung mit einer Spiralfeder ausgestattet, es ist vorteilhaft wenn diese mit einem veränderlichen Druck beaufschlagbar ist, der vom Operateur mittels einem, mit einem Kraftmesser verbundenen Werkzeug kontrolliert einstellbar ist. Die Tatsache, dass die Spiralfeder in der erfindungsgemäßen Vorrichtung auf dem Boden des Außengehäuses, welches zylinderförmig ausgebildet ist, aufliegt, bewirkt vorteilhafterweise eine gute mechanische Stabilität bei einer Beaufschlagung mit mittleren oder höheren Druckkräften während der Implantation.

Ferner ist es von Vorteil, wenn die Dämpfungseinrichtung eine im Außenkörper axial bewegliche Spannhülse aufweist, die sich bei Kniebeuge- und Kniestreckbewegungen nach proximal bewegt und dabei die Feder weiter zusammendrückt, so dass die Kniebewegungen gedämpft werden.

Erfindungsgemäß umfasst die Spiralfeder das proximale Ende der Spannhülse und liegt die Spiralfeder auf dem Flansch der Spannhülse auf. Dadurch wird eine translatorische Bewegung der Spiralfeder im Inneren des Außenkörpers verhindert, da beide Enden der Spiralfeder auf einer formstabilen Fläche gelagert sind.

Außerdem ist es vorteilhaft, wenn der Flansch mit einem Innengewinde ausgestattet ist. Durch die Oberflächenstruktur des Innengewindes erfährt der hauptsächlich im Flansch festgeklemmte Faden zusätzlichen Halt.

Darüber hinaus ist das Befestigungselement der erfindungsgemäßen Vorrichtung als Kegel ausgeführt So ist gewährleistet, dass auch der Kegel mit dem aufgewickelten Faden ohne Materialbelastungen innerhalb der Spannhülse, insbesondere innerhalb des Übergangsbereichs zu seinem Flansch untergebracht ist.

Zweckmäßigerweise ist der Kegel mit seinem distalen Ende an einem Schraubenfortsatz einer Schraube befestigt, wobei die Schraube mit ihrem Außengewinde in das Innengewinde der Spannhülse einzuschrauben ist, wodurch sich vorteilhafterweise der Faden kontrolliert auf den Kegel aufwickeln und sich gleichzeitig verklemmen kann. Ferner ergibt sich daraus der Vorteil, dass die Kraft, mit welcher die Schraube eingedreht wird, in einer definierten Beziehung zur Spannkraft auf den Faden steht, wobei diese Beziehung natürlich dem Anwender bzw. dem Operateur bekannt ist.

Gemäß vorteilhafter Ausgestaltung weist der Schraubenkopf der Schraube eine Aussparung auf, welche in der Form eines Mehrecks bzw. eines Sechsecks ausgebildet ist, wodurch ein Abrutschen des Werkzeugs bei der Beaufschlagung mit einer Zugkraft vorteilhafterweise verhindert ist.

Erfindungsgemäß besteht der Faden der erfindungsgemäßen Vorrichtung aus einem elastischen Material, wodurch eine gleichmäßige Zugbelastung möglich ist und ein Reißen des Fadens verhindert ist. Um diese Eigenschaften zu verstärken, ist in einem Ausführungsbeispiel noch ein zusätzlicher Körper am distalen Ende des elastischen Fadens vorgesehen, wobei der elastische Körper vorzugsweise aus einem Polymer bzw. aus einem Elastomer besteht. Ferner ist ein elastisches Material für den Faden bzw. den Körper von Vorteil, da dadurch die Zugkräfte auf das rekonstruierte vordere Kreuzband entlastet und kompensiert werden.

Das erfindungsgemäße System für eine kontrollierte Beanspruchung bzw. Belastung des rekonstruierten vorderen Kreuzbandes während der Heilungsphase besteht vorteilhafterweise aus der erfindungsgemäßen Vorrichtung (isotonische Kniegelenkschraube) zur Implantation in einen Knochen aus zumindest einem Faden, der als temporärer Ersatz für das rekonstruierte vordere Kreuzband vorgesehen ist, sowie aus einem Halteelement für den zumindest einen Faden, wodurch die Spannung im Faden aufgebaut wird.

Es ist von Vorteil, wenn die Kniegelenkschraube in einen distalen Knochen eines Gelenks eingesetzt wird, so dass die Richtung der beaufschlagten Zugkraft bzw. Zugspannung vom Gelenk weg verläuft. Dadurch ist vorteilhafterweise auch das heilende, körpereigene vordere Kreuzband entlastet.

Außerdem ist es von Vorteil, wenn das erfindungsgemäße System davon ausgeht, dass die Vorrichtung unter einem spitzen Winkel im Bezug auf die Knochenoberfläche in den Knochen eingeschraubt wird, wodurch ihre stabile Verankerung gewährleistet ist, die durch eine Belastung mit Zugkräften nicht destabilisiert wird. Die Richtung des spitzen Winkels innerhalb des Knochens entspricht weitgehend dem Verlauf des vorderen Kreuzbands im Inneren des Kniegelenks.

Außerdem ist es von Vorteil, dass nach einer Montage des erfindungsgemäßen Systems das Kniegelenk während der Heilungsphase in der hinteren Schublade fixiert bleibt und eventuell auftretende Schläge bei den täglichen Mobilisierungsübungen oder unkontrollierte Bewegungen während des Schlafes durch die Spiralfeder aufgefangen werden.

Ein weiterer Vorteil ist, dass der operative Eingriff für die Montage des erfindungsgemäßen Systems minimal invasiv erfolgt und nur zwei gewöhnliche Artroskopieschnitte dazu nötig sind.

Ferner ist es vorteilhaft, dass das körpereigene Kreuzband erhalten bleibt und nicht mehr entfernt werden muss. Dadurch bleibt auch die Propriozeptivität bestehen, so dass die Bandstruktur nach dem Heilungsprozess nicht mehr avital ist. Außerdem ist keine Fremdtransplantatentnahme mehr nötig, so dass sich vorteilhafterweise die Dauer des operativen Eingriffs praktisch halbiert und die Wahrscheinlichkeit einer Komplikation signifikant gesenkt ist. Dadurch ist wiederum der Aufenthalt des mit dem erfindungsgemäßen System versorgten Patienten ebenfalls deutlich verkürzt.

Ausführungsbeispiele der vorliegenden Erfindung werden nachfolgend beschrieben. Sowohl der Aufbau als auch die Anwendung der Erfindung sowie deren Vorteile und Aufgaben sind am besten anhand der folgenden Beschreibung in Verbindung mit der dazugehörigen Zeichnung verständlich. In der Zeichnung zeigt:
- Fig. 1: eine anteriore Ansicht eines menschlichen Knies in Flexionsstellung mit einem ersten Ausführungsbeispiel der erfindungsgemäßen Vorrichtung;
- Fig. 2: eine anteriore Ansicht eines menschlichen Knies in Flexionsstellung mit einem ersten Ausführungsbeispiel des erfindungsgemäßen Systems für eine kontrollierte Beanspruchung eines vorderen Kreuzbands;
- Fig. 3: ein gebeugtes menschliches Knie, an welchem eine Anwendung der erfindungsgemäßen Vorrichtung und des erfindungsgemäßen Systems sinnvoll ist;
- Fig. 3a: eine Vergrößerung des in Fig. 3 eingezeichneten Bildausschnitts;
- Fig. 4: eine Schnittdarstellung eines distalen Endes eines Femurknochens in Flexionsstellung mit dem vorderen Kreuzband und dem hinteren Kreuzband und dem Verlauf des zumindest einen Fadens des erfindungsgemäßen Systems innerhalb der medialen (inneren) Kondyle;
- Fig. 5: eine Draufsicht auf einen Kniegelenkspalt mit den beiden Menisken sowie mit dem vorderen Kreuzband und dem hinteren Kreuzband;
- Fig. 6: einen parallel zur Sagittalebene liegenden, schematisierten Schnitt durch ein menschliches Kniegelenk, an welchem das erfindungsgemäße System zur Anwendung kommt, und
- Fig. 7: eine geschnittene Darstellung der erfindungsgemäßen Vorrichtung zur Implantation in einen Knochen.

Einander entsprechende Teile sind in allen Figuren mit den gleichen Bezugszeichen versehen.

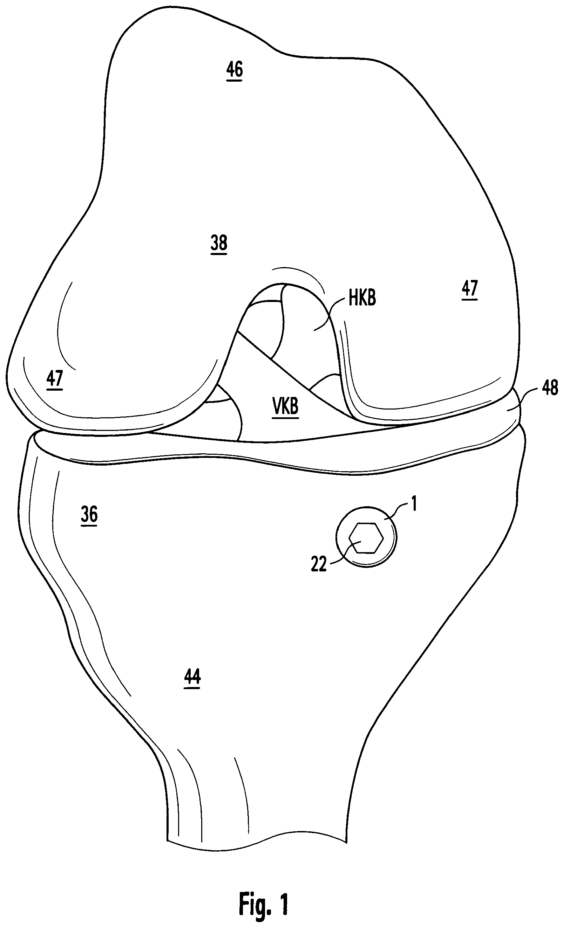

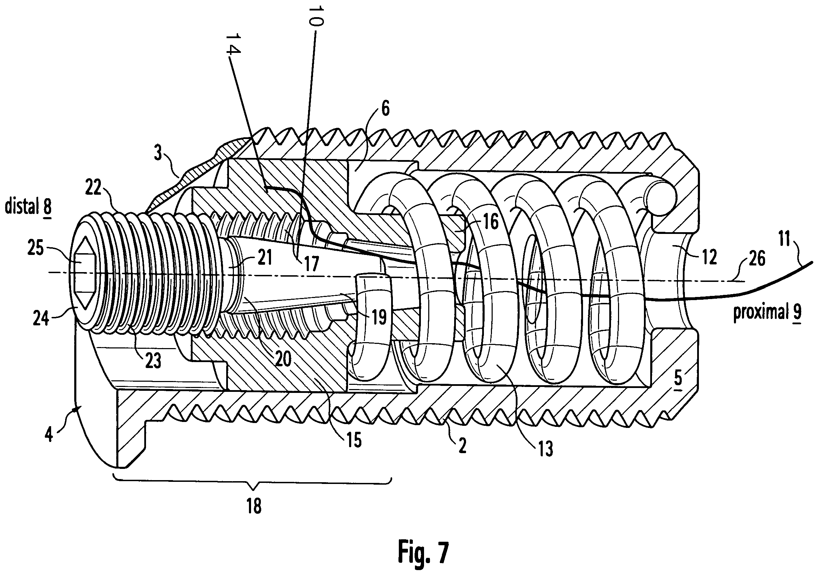

Fig. 1 zeigt eine anteriore Ansicht eines menschlichen Kniegelenks in Flexionsstellung mit einem ersten Ausführungsbeispiel der erfindungsgemäßen Vorrichtung 1 zur Implantation in einen Knochen, wobei dieses Ausführungsbeispiel der erfindungsgemäße Vorrichtung 1 einen zylinderförmigen mit einem Außengewinde 2 versehenen Außenkörper 3 mit einer Stirnseite 4 und mit einem Boden 5 aufweist. Der zylinderförmige Außenkörper 3 ist zwischen seinem distalen Ende 8 und seinem proximalen Ende 9 in seinem Inneren 6 mit einer Dämpfungseinrichtung 7 ausgestattet, wobei innerhalb der Dämpfungseinrichtung 7 zumindest ein Befestigungselement 10 angeordnet ist, welches zumindest einen Faden 11 fixiert, der am proximalen Ende 9 der Vorrichtung 1 durch eine Aussparung 12 im Boden 5 aus dem zylinderförmigen Außenkörper 3 heraus geführt ist, was in Fig. 7 gut zu erkennen ist.

Die erfindungsgemäße Vorrichtung 1 ist unter einem spitzen Winkel a, bezogen auf eine Tangentiale an eine Knochenoberfläche in eine Bohrung, welche in distalproximaler Richtung verläuft, in einen Knochen einschraubbar. Der spitze Winkel α liegt im Bereich von 40° bis 50°, beträgt aber bevorzugt 45°. In diesem ersten Ausführungsbeispiel handelt es sich um ein menschliches Kniegelenk, wobei die erfindungsgemäße Vorrichtung 1 in eine Bohrung im proximalen Ende des Tibiaknochens 44 eingeschraubt wird. Die Position 45 der Bohrung liegt unterhalb des vorderen Kreuzband-Ansatzes lateralanterior.

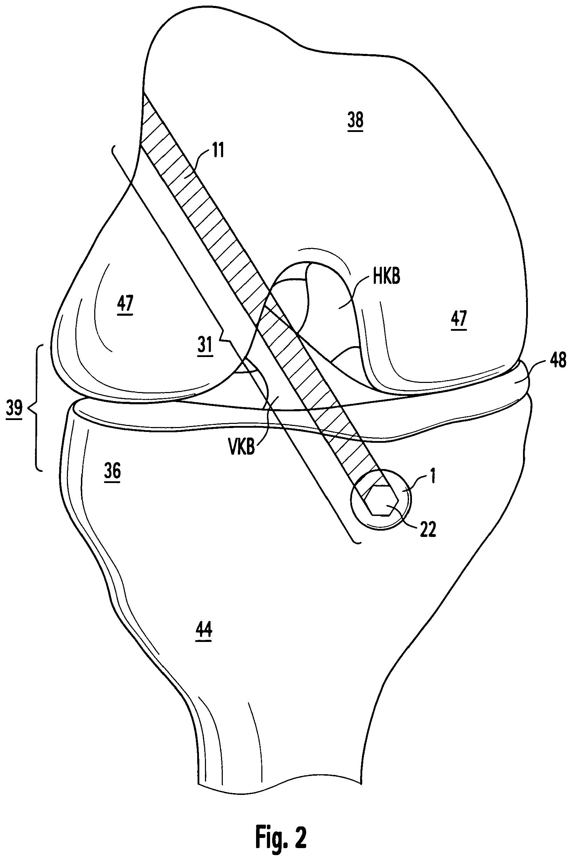

Fig. 2 zeigt eine anteriore Ansicht eines menschlichen Kniegelenks in Flexionsstellung mit einem Ausführungsbeispiel des erfindungsgemäßen Systems für eine kontrollierte Beanspruchung bzw. Belastung eines vorderen Kreuzbands VKB. Das erfindungsgemäße System 31 für eine kontrollierte Beanspruchung bzw. Belastung des beispielsweise durch Nähen rekonstruierten vorderen Kreuzbandes VKB besteht aus einer Vorrichtung 1 zur Implantation in einen Knochen, aus zumindest einem Faden 11 als Ersatz für das rekonstruierte vordere Kreuzband VKB sowie aus einem Halteelement 32 für den zumindest einen Faden 11 und wird bevorzugt während der Heilungsphase eines gerissenen und wieder zusammengefügten Bandes zu dessen Entlastung angewandt.

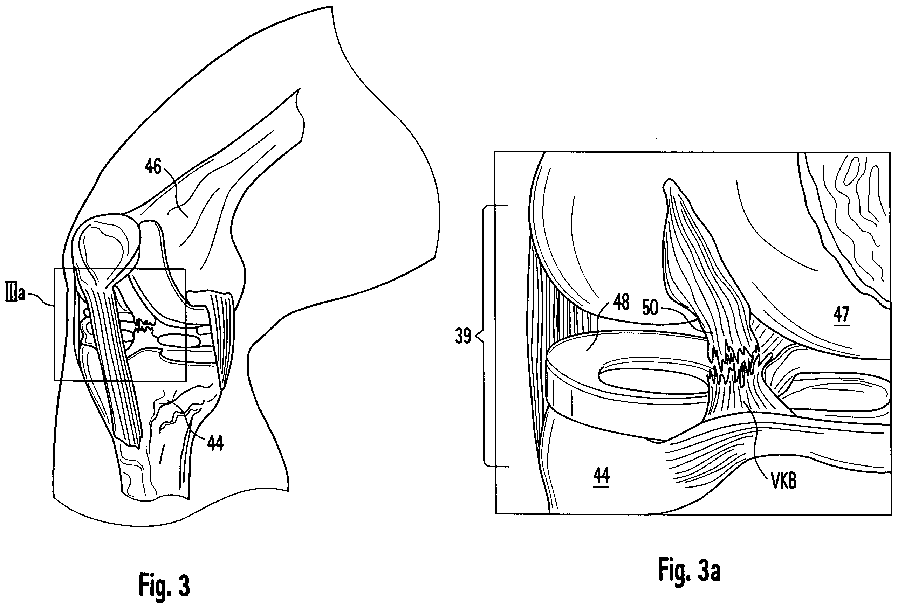

In Fig. 3 ist ein gebeugtes menschliches Knie dargestellt, für welches eine Anwendung der erfindungsgemäßen Vorrichtung und des erfindungsgemäßen Systems bevorzugt vorgesehen ist.

Fig. 3a zeigt eine Vergrößerung des in Fig. 3 eingezeichneten Bildausschnitts mit einer vorderen Kreuzbandruptur 50. Das gerissene vordere Kreuzband VKB wird durch geeignete Maßnahmen wieder rekonstruriert, wobei in der anschließenden Heilungsphase das rekonstruierte vordere Kreuzband VKB nur sehr wenig belastet werden darf, damit das gerissene vordere Kreuzband VKB wieder so zusammenwächst, dass es später seine Funktion wieder voll erfüllen kann. Dadurch, dass das erfindungsgemäße System 31, bestehend aus einer Vorrichtung 1 mit Dämpfungseinrichtung 7, aus zumindest einem Faden 11 sowie aus einem Halteelement 32 bei der in Fig. 3 gezeigten Verletzung eingesetzt wird, wird das rekonstruierte vordere Kreuzband VKB sogar bei kleinen Bewegungen nicht belastet, da durch das erfindungsgemäße System 31, insbesondere durch den zumindest einen Faden 11, der von der Tibia bis zum Femur mit einer definierten, vom Operateur optimierten Kraft von ungefähr 120N gespannt ist, die Funktion des zusammenwachsenden vorderen Kreuzbands VKB komplett übernimmt.

Die Implantation der erfindungsgemäßen Vorrichtung 1 ist in einen Knochen 36 auf einer distalen Seite eines Gelenks vorgesehen. In dem in Fig. 3 bzw. 3a gezeigten Anwendungsfall ist das das proximale Ende eines Tibiaknochens 44, welcher an das menschliche Kniegelenk bzw. an dessen Gelenkspalt 39 grenzt.

Der zumindest eine Faden 11, welcher während des Heilungsprozesses die Funktion des zu entlastenden vorderen Kreuzbands VKB übernimmt, ist über die erfindungsgemäße Vorrichtung 1, welche Bestandteil des ebenfalls erfindungsgemäßen Systems 31 ist, mit einer veränderlichen Zugspannung beaufschlagbar, so dass der Operateur bei einer Implementierung des erfindungsgemäßen Systems 31 nach der Rekonstruktion des vorderen Kreuzbandes VKB bzw. nach der Rekonstruktion eines Bandes im Allgemeinen die Zugspannung optimal anpassen kann. Dadurch ist gewährleistet, dass das erfindungsgemäße System 31 die Funktion des rekonstruierten Bandes bzw. die Funktion des rekonstruierten vorderen Kreuzbandes VKB während seiner Heilungsphase übernimmt.

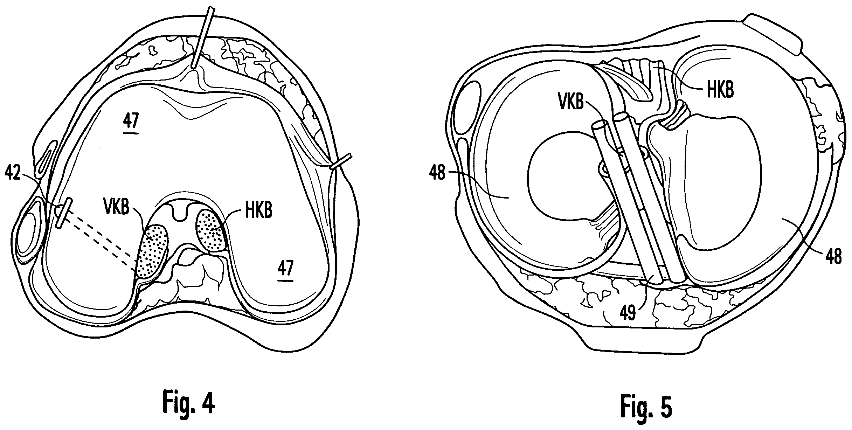

Fig. 4 zeigt eine Schnittdarstellung eines distalen Endes eines Femurknochens 46 bzw. eine Schnittdarstellung beider Kondylen 47 am menschlichen Kniegelenk in Flexionsstellung. An den Innenseiten der beiden Kondylen 47 sind das vordere Kreuzband VKB sowie das hintere Kreuzband HKB angewachsen. Des Weiteren ist in Fig. 4 das erfinderische System 31 mit dem Halteelement 32 und dem zumindest einem Faden 11 schematisch eingezeichnet, wobei Halteelement 32 und Faden 11, welcher gestrichelt dargestellt ist, in die Zeichenebene projiziert sind. Innerhalb des menschlichen Kniegelenks verläuft der zumindest eine Faden 11 durch einen ersten Knochentunnel 35 innerhalb des Tibiaknochens 44 über den Gelenkspalt 39 in einen zweiten Knochentunnel 37 innerhalb einer Kondyle 47, wobei die Austrittsöffnung des zweiten Knochentunnels 37 lateral bzw. außen an dieser Kondyle 47 liegt und der zumindest eine Faden 11 weitgehend parallel zum rekonstruierten vorderen Kreuzband VKB gespannt ist.

Fig. 5 zeigt eine geschnittene Draufsicht auf einen Kniegelenkspalt 39 mit den beiden Menisken 48 sowie mit dem vorderen Kreuzband VKB und dem hinteren Kreuzband HKB von proximal bzw. von oben aus betrachtet. Parallel zu dem vorderen Kreuzband VKB verläuft bei diesem Ausführungsbeispiel der Erfindung ein Doppelfaden 49 zur Entlastung des darunter liegenden rekonstruierten vorderen Kreuzbands VKB.

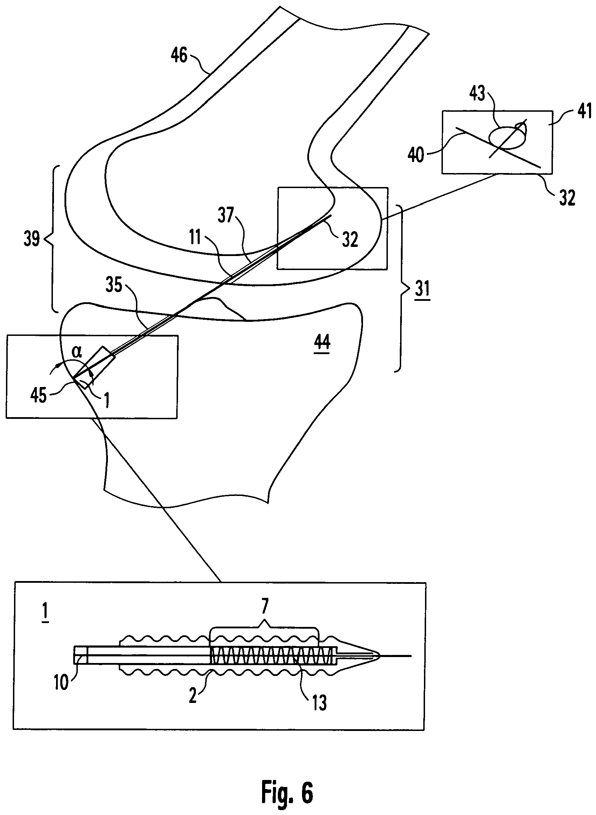

Fig. 6 zeigt einen parallel zur Sagittalebene liegenden, schematisierten Schnitt durch ein isotonisch in einer vorderen Schublade gehaltenes menschliches Kniegelenk, an welchem das erfindungsgemäße System 31, welches ein Halteelement 32, zumindest einen Faden 11 sowie die erfindungsgemäßen Vorrichtung 1 umfasst, zur Anwendung kommt. Das erfindungsgemäße System 31 besteht in diesem Ausführungsbeispiel aus der bereits beschriebenen Vorrichtung 1 zur Implantation in einen Knochen, bevorzugt in das proximale Ende eines menschlichen Tibiaknochens 44, aus zumindest einen Faden 11 als funktionaler Ersatz bzw. als Unterstützung für das rekonstruierte Band bzw. vordere Kreuzband VKB eines menschlichen Kniegelenks sowie aus einem Haltelement 32, welches den zumindest einen Faden 11 an einer Oberfläche eines proximalen Knochens eines Gelenks, insbesondere eines Kniegelenks während der Heilungsphase des rekonstruierten Bandes befestigt. In Fig. 6 ist gezeigt, dass der zumindest eine Faden 11 innerhalb eines ersten Knochentunnels 35 in einem distalen Knochen 36 eines Gelenks, insbesondere eines Kniegelenks, sowie innerhalb eines zweiten Knochentunnels 37 innerhalb eines proximalen Knochens 38 des Gelenks bzw. des Kniegelenks verläuft, wobei der Faden 11 bzw. mehrere parallel angeordnete Fäden 49 über den Gelenkspalt bzw. über den Kniegelenkspalt 39 gespannt sind. Der zumindest eine Faden 11 tritt aus dem zweiten Knochentunnel 38 aus und ist mittels des Halteelements 32 an einem proximalen Ende außerhalb des zweiten Knochentunnels 37 fixiert. Das Halteelement 32 besteht aus einem Metallplättchen 40 und einem Stopper 41 und liegt auf dem proximalen Ende des zweiten Knochentunnels 37 auf, wobei der Stopper 41, welcher als Knoten 42 in dem Faden 11 bzw. als gemeinsamer Knoten aller Fäden 49 ausgebildet ist, den Faden 11 bzw. die Fäden 49 am Metallplättchen 40 fixiert. Der Stopper 41 kann auch als Perle 43 ausgebildet ist, wobei ein Ende des zumindest einen Fadens 11 mit der Perle 43 verschweißt ist und die Perle 43 selbst aus einem biokompatiblen Material gefertigt ist.

Fig. 7 zeigt eine geschnittene Darstellung der erfindungsgemäßen Vorrichtung 1 zur Implantation in einen Knochen. Die erfindungsgemäße Vorrichtung 1 umfasst einen zylinderförmigen Außenkörper 3, welcher mit einem Außengewinde 2 versehen ist, um in einen Knochen 44 bzw. in Knochengewebe eingeschraubt zu werden. Dabei verankert sich der zylinderförmige Außenkörper selbst im Knochengewebe, indem dieses insbesondere an der Oberfläche seines Außengewebes gut anwächst. Der zylinderförmige Außenkörper 3 weist eine Stirnseite 4 und einen Boden 5 auf, wobei die Stirnseite 4 nach dem Einbringen der erfindungsgemäßen Vorrichtung 1 in den Knochen zur Knochenoberfläche hin orientiert ist. Im Inneren 6 der erfindungsgemäßen Vorrichtung 1 bzw. des zylinderförmigen Außenkörpers 3 ist eine Dämpfungseinrichtung 7 zwischen einem distalen Ende 8 und einem proximalen Ende 9 untergebracht, wobei die Dämpfungseinrichtung 7 mindestens ein Befestigungselement 10 aufweist, welches als Kegel 19 ausgeführt ist und am distalen Ende 8 zumindest einen Faden 11 fixiert, welcher größtenteils innerhalb einer Dämpfungseinrichtung 7, parallel zu ihrer Längsachse 26 verläuft und am proximalen Ende 9 der Vorrichtung 1 durch eine Aussparung 12 im Boden 5 aus dem zylinderförmigen Außenkörper 3 herausgeführt ist.

Die Dämpfungseinrichtung 7 ist mit einer Spiralfeder 13 ausgestattet, welche mit einem vom Operateur veränderbaren Druck beaufschlagt ist und an ihrem proximalen Ende 9 auf dem Boden 5 des zylinderförmigen Außenkörpers 3 aufliegt. Ferner ist innerhalb der Dämpfungsvorrichtung 7 eine Hülse bzw. eine Spannhülse 14 angeordnet, welche einen Flansch 15 mit einem Innengewinde 17 aufweist, auf welchem die Spiralfeder 13 mit ihrem distalen Ende 8 aufliegt. Das proximale Ende 16 der Hülse 14 ist in die Spiralfeder 13 hineingeschoben, so dass diese die Hülse 14 im Bereich der distalen Hälfte 18 des zylinderförmigen Außenkörpers 3 umgibt. Das Befestigungselement 10 bzw. der Kegel 19 ist mit seinem distalen Ende 20 an einem Schraubenfortsatz 21 einer Schraube 22 befestigt, wobei die Schraube 22 für ein kontrolliertes Aufwickeln des Fadens 11 auf den Kegel 19 vorgesehen ist, wodurch sich die Fadenspannung erhöht. Die Schraube 22, dessen Schraubenkopf 24 eine Aussparung 25 in Form eines Mehrecks, bevorzugt eines Sechsecks aufweist, wird dabei mit ihrem Außengewinde 23 in das Innengewinde 17 des Flansches 15 eingeschraubt.

Entsprechend dem erfindungsgemäßen Ausführungsbeispiel der erfindungsgemäßen Vorrichtung 1 weist die Dämpfungseinrichtung 7 einen Faden 11 aus einem elastischen Material auf, wobei der Faden mittels Verklemmung an dem zumindest einen Befestigungselement 10 befestigt ist. Die Verklemmung des elastischen Fadens 11 wird dadurch erzielt, dass dieser auf den Kegel 19 aufgewickelt wird und somit an der Innenwandung des proximalen Endes 16 der Spannhülse 14 und dem Kegel 19 eingeklemmt ist.

Eine erfindungsgemäße Vorrichtung 1 mit einer Dämpfungseinrichtung 7 sowie einem elastischen Faden 11 an dessen distalen Ende ein elastischer Körper, bestehend aus einem Polymer oder einem Elastomer, der zumindest einen Faden oder mehrere Fäden umgibt, stellt ein zusätzliches Ausführungsbeispiel der vorliegenden Erfindung dar. Die beiden letztgenannten Ausführungsbeispiele sind jedoch nicht in der Zeichnung dargestellt.

Die Erfindung ist nicht auf das in der Zeichnung dargestellte Ausführungsbeispiel, insbesondere nicht auf eine Anwendung für ein Kniegelenk beschränkt. Eine Anwendung der Erfindung als ein temporärer funktionaler Ersatz eines Schultergelenks oder eines anderen Gelenks ist ebenfalls möglich. Alle vorstehend beschriebenen und in der Zeichnung dargestellten Merkmale sind beliebig miteinander kombinierbar.

## Patentansprüche

1. Vorrichtung (1) zur Implantation in einen Knochen, die einen mit einem Außengewinde (2) versehenen Außenkörper (3) mit einer Stirnseite (4) und mit einem Boden (5) aufweist, wobei der Außenkörper (3) in seinem Inneren (6) eine Dämpfungseinrichtung (7) zwischen einem distalen Ende (8) und einem proximalen Ende (9) aufweist und wobei innerhalb der Dämpfungseinrichtung (7) zumindest ein Befestigungselement (10) angeordnet ist, welches zumindest einen Faden (11) fixiert, der am proximalen Ende (9) der Vorrichtung (1) durch eine Aussparung (12) im Boden (5) aus dem Außenkörper (3) heraus geführt ist und wobei die Dämpfungseinrichtung (7) einen Faden (11) aus einem elastischen Material aufweist, wobei der Faden (11) mittels Verklemmung an dem zumindest einen Befestigungselement (10) befestigt ist,
**dadurch gekennzeichnet,**
**dass** die Dämpfungseinrichtung (7) eine Spannhülse (14) mit einem Flansch (15) aufweist und mit einer Spiralfeder (13) ausgestattet ist,
**dass** die Spiralfeder (13) ein proximales Ende (16) der Spannhülse (14) umfasst und auf ihrem Flansch (15) aufliegt, und
**dass** das Befestigungselement (10) als Kegel (19) ausgeführt ist.

2. Vorrichtung nach Anspruch 1
**dadurch gekennzeichnet,**
**dass** die Spiralfeder (13) mit einem veränderlichen Druck beaufschlagt ist und an ihrem proximalen Ende (9) auf dem Boden (5) des Außenkörpers (3) aufliegt.

3. Vorrichtung nach Anspruch 1,
**dadurch gekennzeichnet,**
**dass** der Flansch (15) mit einem Innengewinde (17) ausgestattet ist

4. Vorrichtung nach einem der Ansprüche 1 und 3,
**dadurch gekennzeichnet,**
**dass** die Spannhülse (14) mit ihrem Flansch (15) im Bereich der distalen Hälfte (18) des Außenkörpers (3) angeordnet ist.

5. Vorrichtung nach Anspruch 3,
**dadurch gekennzeichnet,**
**dass** die Länge des Kegels (19) des Befestigungselements (10) in etwa der Länge des Innengewindes (17) des Flansches (15) entspricht.

6. Vorrichtung nach Anspruch 5,
**dadurch gekennzeichnet,**
**dass** der Kegel (19) mit seinem distalen Ende (20) an einem Schraubenfortsatz (21) einer Schraube (22) befestigt ist.

7. Vorrichtung nach Anspruch 6,
**dadurch gekennzeichnet,**
**dass** die Schraube (22) mit ihrem Außengewinde (23) in das Innengewinde (17) des Flansches (15) der Spannhülse (14) einzuschrauben ist.

8. Vorrichtung nach Anspruch 7,
**dadurch gekennzeichnet,**
**dass** ein Schraubenkopf (24) der Schraube (22) eine Aussparung (25) in Form eines Mehrecks oder eines Sechsecks aufweist.

9. Vorrichtung nach einem der Ansprüche 2 oder 3 bis 8,
**dadurch gekennzeichnet,**
**dass** der zumindest eine Faden (11) größtenteils innerhalb der Spiralfeder (13), parallel zu ihrer Längsachse (26) verläuft.

10. Vorrichtung nach einem der Ansprüche 1 bis 9,
**dadurch gekennzeichnet,**
**dass** die Dämpfungseinrichtung (7) einen Faden (11) aus einem elastischen Material aufweist, wobei an dem elastischen Faden an dessen distalen Ende ein elastischer Körper vorgesehen ist, der zumindest einen Faden (11) oder mehrere Fäden umgibt.

11. Vorrichtung nach Anspruch 10,
**dadurch gekennzeichnet,**
**dass** der elastische Körper ein Polymer oder ein Elastomer ist.

12. Vorrichtung nach einem der Ansprüche 1 bis 8,
**dadurch gekennzeichnet,**
**dass** die Vorrichtung (1) unter einem spitzen Winkel im Bereich von 40° bis 50°, bezogen auf eine Tangentiale an eine Knochenoberfläche, in eine Bohrung in einen Knochen einschraubbar ist.

13. Vorrichtung nach einem der Ansprüche 1 bis 12,
**dadurch gekennzeichnet,**
**dass** der Außenkörper zylinderförmig ist.

14. System (31) für eine kontrollierte Beanspruchung des aus den beiden Faserbündeln rekonstruierten vorderen Kreuzbandes (VKB) während der Heilungsphase, welches eine Vorrichtung (1) zur Implantation in einen Knochen nach einem der Ansprüche 1 bis 13, zumindest einen Faden (11) als Ersatz für das rekonstruierte vordere Kreuzband (VKB) fixiert, sowie ein Halteelement (32) für den zumindest einen Faden (11) aufweist.

15. System nach Anspruch 14,
**dadurch gekennzeichnet,**
**dass** eine Implantation der Vorrichtung (1) in einen Knochen auf einer distalen Seite eines Gelenks vorgesehen ist.

16. System nach Anspruch 14 oder 15,
**dadurch gekennzeichnet,**
**dass** die Vorrichtung (1) unter einem spitzen Winkel (α) im Bereich von 40° bis 50°, bezogen auf eine Tangentiale an eine Knochenoberfläche, in eine Bohrung in einen Knochen einschraubbar ist.

17. System nach einem der Ansprüche 14 bis 16,
**dadurch gekennzeichnet,**
**dass** der zumindest eine Faden (11) über die Vorrichtung (1) mit einer veränderlichen Zugspannung beaufschlagbar ist.

18. System nach einem der Ansprüche 14 bis 17,
**dadurch gekennzeichnet,**
**dass** der zumindest eine Faden (11) zum einen innerhalb eines ersten Knochentunnels (35) verläuft, wobei sich der erste Knochentunnel (35) an die Bohrung in einen distalen Knochen (36) anschließt als auch innerhalb eines zweiten Knochentunnels (37) in einen proximalen Knochen (38) eines Gelenkes verläuft und einen Gelenkspalt (39) überbrückt.

19. System nach Anspruch 18,
**dadurch gekennzeichnet,**
**dass** der zumindest ein Faden (11) aus dem zweiten Knochentunnel (37) austritt und mittels des Halteelements (32) an einem proximalen Ende des zweiten Knochentunnels (37) fixiert ist.

20. System nach Anspruch 19,
**dadurch gekennzeichnet,**
**dass** das Halteelement (32) aus einem Metallplättchen (40) und einem Stopper (41) besteht, wobei das Metallplättchen (40) auf dem proximalen Ende des zweiten Knochentunnels (37) aufliegt und der Stopper (41) den zumindest einen Faden (11) am Metallplättchen (40) fixiert.

21. System nach Anspruch 20,
**dadurch gekennzeichnet,**
**dass** der Stopper (41) als Knoten (42) in dem Faden (11) bzw. als gemeinsamer Knoten (42) aller Fäden (11) ausgebildet ist.

22. System nach Anspruch 21,
**dadurch gekennzeichnet,**
**dass** der Stopper (41) als Perle (43) ausgebildet ist, wobei ein Ende des zumindest einen Fadens (11) mit der Perle (43) verschweißt oder verknotet ist.

## Claims

1. Device (1) for implantation into a bone, which device comprises an outer body (3) which is provided with an external thread (2) and has a front end (4) and a bottom end (5), wherein the outer body (3) has in its interior (6) a damping means (7) between a distal end (8) and a proximal end (9) and wherein arranged within the damping means (7) there is at least one fastening element (10) which fixes at least one thread (11) which at the proximal end (9) of the device (1) is guided through a recess (12) in the bottom end (5) and out of the outer body (3) and wherein the damping means (7) comprises a thread (11) made of an elastic material, wherein the thread (11) is fastened by means of clamping to the at least one fastening element (10),
**characterised in that**
the damping means (7) comprises a clamping sleeve (14) with a flange (15) and is provided with a spiral spring (13),
**in that** the spiral spring (13) fits around a proximal end (16) of the clamping sleeve (14) and rests on its flange (15), and
**in that** the fastening element (10) is embodied in the form of a cone (19).

2. Device according to claim 1,
**characterised in that**
the spiral spring (13) is subjected to a variable pressure and at its proximal end (9) rests on the bottom end (5) of the outer body (3).

3. Device according to claim 1,
**characterised in that**
the flange (15) is provided with an internal thread (17).

4. Device according to one of claims 1 and 3,
**characterised in that**
the clamping sleeve (14) is arranged with its flange (15) in the region of the distal half (18) of the outer body (3) .

5. Device according to claim 3,
**characterised in that**
the length of the cone (19) of the fastening element (10) corresponds roughly to the length of the internal thread (17) of the flange (15).

6. Device according to claim 5,
**characterised in that**
the cone (19) is fastened with its distal end (20) to a threaded extension (21) of a screw (22).

7. Device according to claim 6,
**characterised in that**
the screw (22) has to be screwed by its external thread (23) into the internal thread (17) of the flange (15) of the clamping sleeve (14).

8. Device according to claim 7,
**characterised in that**
a screw head (24) of the screw (22) comprises a recess (25) in the form of a polygon or a hexagon.

9. Device according to one of claims 2 or 3 to 8,
**characterised in that**
the at least one thread (11) runs for the most part within the spiral spring (13), parallel to its longitudinal axis (26).

10. Device according to one of claims 1 to 9,
**characterised in that**
the damping means (7) comprises a thread (11) made of an elastic material, wherein provided at the distal end of the elastic thread there is an elastic body which fits around at least one thread (11) or a plurality of threads.

11. Device according to claim 10,
**characterised in that**
the elastic body is a polymer or an elastomer.

12. Device according to one of claims 1 to 8,
**characterised in that**
the device (1) can be screwed into a hole drilled in a bone at an acute angle in the range of from 40° to 50°, related to a tangent to a bone surface.

13. Device according to one of claims 1 to 12,
**characterised in that**
the outer body is cylindrical in shape.

14. System (31) for controlled loading of the anterior cruciate ligament (ACL) reconstructed from the two fibre bundles during the healing phase, in which a device (1) for implantation into a bone according to one of claims 1 to 13, fixes at least one thread (11) as a replacement for the reconstructed anterior cruciate ligament (ACL), and comprises a retaining element (32) for the at least one thread (11).

15. System according to claim 14,
**characterised in that**
provision is made for implantation of the device (1) into a bone on a distal side of a joint.

16. System according to claim 14 or 15,
**characterised in that**
the device (1) can be screwed into a hole drilled in a bone at an acute angle (α) in the range of from 40° to 50°, related to a tangent to a bone surface.

17. System according to one of claims 14 to 16,
**characterised in that**
the at least one thread (11) can be subjected to a variable tensile loading by means of the device (1).

18. System according to one of claims 14 to 17,
**characterised in that**
the at least one thread (11) runs firstly within a first bone tunnel (35), wherein the first bone tunnel (35) adjoins the hole drilled in a distal bone (36), and also runs within a second bone tunnel (37) in a proximal bone (38) of a joint and bridges a joint gap (39).

19. System according to claim 18,
**characterised in that**
the at least one thread (11) emerges from the second bone tunnel (37) and is fixed by means of the retaining element (32) at a proximal end of the second bone tunnel (37) .

20. System according to claim 19,
**characterised in that**
the retaining element (32) comprises a small metal plate (40) and a stopper (41), wherein the small metal plate (40) rests on the proximal end of the second bone tunnel (37) and the stopper (41) fixes the at least one thread (11) to the small metal plate (40).

21. System according to claim 20,
**characterised in that**
the stopper (41) is embodied in the form of a knot (42) in the thread (11) or of a common knot (42) of all the threads (11).

22. System according to claim 21,
**characterised in that**
the stopper (41) is embodied in the form of a bead (43), wherein one end of the at least one thread (11) is welded or knotted with the bead (43).

## Revendications

1. Dispositif (1) à implanter dans un os, qui comprend un corps extérieur (3) pourvu d'un filet extérieur (2) avec une face frontale (4) et un fond (5), dans lequel l'intérieur (6) du corps extérieur (3) présente un système d'amortissement (7) entre une extrémité distale (8) et une extrémité proximale (9) et dans lequel au moins un élément de fixation (10) est agencé à l'intérieur du système d'amortissement (7), lequel fixe au moins un fil (11) qui est guidé par l'extrémité proximale (9) du dispositif (1) à l'extérieur du corps extérieur (3) en passant par un évidement (12) ménagé dans le fond (5) et dans lequel le système d'amortissement (7) présente un fil (11) composé d'un matériau élastique, dans lequel le fil (11) est fixé par serrage au au moins un élément de fixation (10),
**caractérisé**
**en ce que** le système d'amortissement (7) présente une douille de serrage (14) pourvue d'une bride (15) et est équipé d'un ressort spiral (13),
**en ce que** le ressort spiral (13) entoure une extrémité proximale (16) de la douille de serrage (14) et repose sur sa bride (15), et
**en ce que** l'élément de fixation (10) est réalisé sous la forme d'un cône (19).

2. Dispositif selon la revendication 1,
**caractérisé**
**en ce que** le ressort spiral (13) est soumis à l'effet d'une pression variable et repose par son extrémité proximale (9) sur le fond (5) du corps extérieur (3).

3. Dispositif selon la revendication 1,
**caractérisé**
**en ce que** la bride (15) est équipée d'un filet intérieur (17).

4. Dispositif selon l'une des revendications 1 et 3,
**caractérisé**
**en ce que** la douille de serrage (14) est agencée par sa bride (15) dans la zone de la moitié distale (18) du corps extérieur (3).

5. Dispositif selon la revendication 3,
**caractérisé**
**en ce que** la longueur du cône (19) de l'élément de fixation (10) correspond sensiblement à la longueur du filet intérieur (17) de la bride (15).

6. Dispositif selon la revendication 5,
**caractérisé**
**en ce que** le cône (19) est fixé par son extrémité distale (20) à une partie saillante (21) d'une vis (22).

7. Dispositif selon la revendication 6,
**caractérisé**
**en ce que** la vis (22) est à visser par son filet extérieur (23) dans le filet intérieur (17) de la bride (15) de la douille de serrage (14).

8. Dispositif selon la revendication 7,
**caractérisé**
**en ce qu'**une tête de vis (24) de la vis (22) présente un évidement (25) se présentant sous la forme d'un polygone ou d'un hexagone.

9. Dispositif selon l'une quelconque des revendications 2 ou 3 à 8,
**caractérisé**
**en ce que** le au moins un fil (11) s'étend en grande partie à l'intérieur du ressort spiral (13), parallèlement à l'axe longitudinal (26) de celui-ci.

10. Dispositif selon l'une quelconque des revendications 1 à 9,
**caractérisé**
**en ce que** le système d'amortissement (7) comprend un fil (11) en matériau élastique, dans lequel un corps élastique qui entoure au moins un fil (11) ou plusieurs fils est prévu sur le fil élastique à l'extrémité distale de celui-ci.

11. Dispositif selon la revendication 10,
**caractérisé**
**en ce que** le corps élastique est un polymère ou un élastomère.

12. Dispositif selon l'une quelconque des revendications 1 à 8,
**caractérisé**
**en ce que** le dispositif (1) peut être vissé dans un trou dans un os en formant un angle aigu dans la plage de 40° à 50°, par rapport à une tangente sur une surface de l'os.

13. Dispositif selon l'une quelconque des revendications 1 à 12,
**caractérisé**
**en ce que** le corps extérieur est cylindrique.

14. Système (31) pour une sollicitation contrôlée du ligament croisé antérieur (VKB) reconstruit à partir des deux faisceaux de fibres pendant la phase de guérison, lequel fixe un dispositif (1) à implanter dans un os selon l'une quelconque des revendications 1 à 13, au moins un fil (11) en tant que prothèse pour le ligament croisé antérieur (VKB) reconstruit, ainsi qu'un élément de retenue (32) pour le au moins un fil (11) .

15. Système selon la revendication 14,
**caractérisé**
**en ce qu'**une implantation du dispositif (1) dans un os est prévue sur une face distale d'une articulation.

16. Système selon la revendication 14 ou 15,
**caractérisé**
**en ce que** le dispositif (1) peut être vissé dans un trou dans un os en formant un angle aigu (α) dans la plage de 40° à 50° par rapport à une tangente sur une surface de l'os.

17. Système selon l'une quelconque des revendications 14 à 16,
**caractérisé**
**en ce que** le au moins un fil (11) peut être soumis à l'effet d'une contrainte de traction variable par le dispositif (1).

18. Système selon l'une quelconque des revendications 14 à 17,
**caractérisé**
**en ce que** le au moins un fil (11) s'étend d'une part à l'intérieur d'un premier tunnel osseux (35), dans lequel le premier tunnel osseux (35) se situe dans le prolongement du trou dans un os distal (36) ainsi qu'à l'intérieur d'un deuxième tunnel osseux (37) dans un os proximal (38) d'une articulation et comble un interligne articulaire (39).

19. Système selon la revendication 18,
**caractérisé**
**en ce que** le au moins un fil (11) sort du deuxième tunnel osseux (37) et est fixé au moyen de l'élément de retenue (32) à une extrémité proximale du deuxième tunnel osseux (37).

20. Système selon la revendication 19,
**caractérisé**
**en ce que** l'élément de retenue (32) est constitué d'une plaquette métallique (40) et d'une butée (41), dans lequel la plaquette métallique (40) repose sur l'extrémité proximale du deuxième tunnel osseux (37) et la butée (41) fixe le au moins un fil (11) à la plaquette métallique (40).

21. Système selon la revendication 20,
**caractérisé**
**en ce que** la butée (41) est réalisée sous la forme d'un noeud (42) dans le fil (11) ou sous la forme d'un noeud (42) commun de tous les fils (11).

22. Système selon la revendication 21,
**caractérisé**
**en ce que** la butée (41) est réalisée sous la forme d'une perle (43), dans lequel une extrémité du au moins un fil (11) est soudée ou nouée à la perle (43).
